# EUROPEAN PATENT SPECIFICATION

(11) **EP 3 346 984 B1**
(45) Date of publication and mention of the grant of the patent: **23.01.2019**
(21) Application number: 16753358.7
(22) Date of filing: 16.08.2016
(51) Int. Cl.: A61K 8/46, A61Q 5/02, A61Q 5/12, A61K 8/81, A61K 8/895

(54) **PERSONAL CLEANSING COMPOSITION**
KÖRPERREINIGUNGSZUSAMMENSETZUNGEN
COMPOSITION D'HYGIENE PERSONNELLE LIQUIDE

(30) Priority: 10.09.2015 EP 15184746
(43) Date of publication of application: 18.07.2018
(73) Proprietor: Unilever PLC, London, Greater London EC4Y 0DY (GB); Unilever N.V., 3013 AL Rotterdam (NL)
(72) Inventor: AINGER, Nicholas, John, Wirral Merseyside CH63 3JW (GB)
(74) Representative: Chisem, Janet
(86) International application number: PCT/EP2016/069448
(87) International publication number: WO 2017/042004

(56) References cited:
- EP-A2- 2 495 232
- WO-A1-2010/121876
- WO-A1-2013/017328
- WO-A1-2013/045376
- WO-A1-2014/082846
- WO-A1-2014/082851

## Description

### Field of the Invention

The present invention relates to personal cleansing compositions such as liquid soaps, body washes and shampoos.

### Background and Prior Art

In personal cleansing compositions such as liquid soaps, body washes and shampoos, the suspension and delivery of benefit agents are often key drivers of product performance. Benefit agents are typically present as dispersed hydrophobic emulsion droplets (such as silicones) or dispersed particulate solids (such as flaky anti-dandruff actives). A problem encountered by the formulator is keeping the dispersed benefit agent suspended and the total product stable while still providing satisfactory rheology as well as detersive and conditioning performance.

Homopolymers and copolymers of acrylic acid have been used as structurants to provide effective thickening as well as effective storage stable suspension of insoluble particles in aqueous surfactant systems. Examples of such structuring polymers include carbomers, alkali swellable emulsion (ASE) polymers and hydrophobically modified alkali swellable emulsion (HASE) polymers.

ASE and HASE polymers provide weight efficient thickening and improved tolerance to electrolytes, allowing improved product appearance (e.g. transparency).

However, the ability of dispersed benefit agents (such as silicones) to efficiently deposit onto hair from ASE or HASE structured systems is severely reduced at low product pH. A mildly acidic pH is often desirable in shampoos for reasons such as reduced hair fibre swelling, improved hair lustre, better compatibility with acidic skin or hair care actives in the formulation and optimized efficacy of certain organic preservative systems.

The present invention addresses this problem. WO2014082851 discloses aqueous cleansing composition comprising silicone conditioning agents.

### Summary of the Invention

The present invention provides a personal cleansing composition having a pH ranging from 3 to 6.5 and comprising, in an aqueous continuous phase:
(i) one or more anionic cleansing surfactants;
(ii) one or more dispersed benefit agents selected from silicones, and
(iii) a structuring polymer selected from alkali swellable emulsion (ASE) polymers and hydrophobically modified alkali swellable emulsion (HASE) polymers;
characterized in that the one or more anionic cleansing surfactants are selected from alkyl ether sulphates of general formula (I):

R-O-(CH₂CH₂-O)ₙ-SO₃⁻M⁺ (I)

in which R is a straight or branched chain alkyl group having 10 to 14 carbon atoms, n is
a number that represents the degree of ethoxylation and ranges from 3 to 3.5, and M is
an alkali metal, ammonium or alkanolammonium cation.

### Detailed Description and Preferred Embodiments

All molecular weights as used herein are weight average molecular weights, unless otherwise specified.

By "aqueous continuous phase" is meant a continuous phase which has water as its basis.

Suitably, the composition of the invention will comprise from about 50 to about 90%, preferably from about 55 to about 85%, more preferably from about 60 to about 85%, most preferably from about 65 to about 83% water (by weight based on the total weight of the composition).

The composition of the invention comprises one or more anionic cleansing surfactants selected from alkyl ether sulphates of general formula (I) defined above.

In general formula (I), M is preferably sodium, potassium, ammonium or ethanolamine, R is preferably a C₁₀ tor C₁₂ n-alkyl group and the average degree of ethoxylation n preferably ranges from 3.0 to 3.2.

Particularly preferred is SLES 3EO (i.e. sodium lauryl ether sulphate in which the average degree of ethoxylation n is 3.0)

Commercially produced alkyl ether sulphates generally contain a mixture of homologues with the actual composition reflecting the aliphatic alcohol feedstock selection and the degree of ethoxylation.

Accordingly, the degree of ethoxylation (n in general formula (I)) is a statistical average value which may be an integer or a fraction. The value of n is governed by the starting molar ratio of ethylene oxide to aliphatic alcohol in the ethoxylation reaction and the temperature, time and catalytic conditions under which the ethoxylation reaction takes place.

A commercially produced alkyl ether sulphate having general formula (I) will usually comprise a mixture of homologues in which from 55 to 80 mol% of the total mixture is made up of homologues with ethoxy chains of 5EO or less (down to 0EO, i.e. unethoxylated alkyl sulphate), with the remainder of the mixture made up of homologues with ethoxy chains of 6EO or more (up to about 10EO). Higher homologues (e.g. up to about 15EO) may also be present on small amounts (typically no more than 1 to 2 mol% of the total mixture per individual homologue). A typical breakdown in molar percentage terms for commercially produced alkyl ether sulphates having general formula (I) is given in the following Table:

| **x** | **% m/m of R-O-(CH₂CH₂-O)x-SO3-M+** |
|---|---|
| **0** | 10 to 15 |
| **1** | 7 to 11 |
| **2** | 10 to 12 |
| **3** | 10 to 15 |
| **4** | 10 to 12 |
| **5** | 9 to 11 |
| **6** | 6 to 10 |
| **7** | 5 to 9 |
| **8** | 3 to 7 |
| **9** | 3 to 5 |
| **10** | 2 to 4 |
| **11** | 0 to 2 |
| **12** | 0 to 2 |
| **13** | 0 to 1 |
| **14** | 0 to 1 |
| **15** | 0 to 0.5 |

Examples of commercially produced alkyl ether sulphates having general formula (I) for use in the invention include STEOL® CS-330 HA (ex Stepan Company) and Texapon® N 70 LS (ex BASF).

In a typical composition according to the invention the level of alkyl ether sulphate of general formula (I) will generally range from 5 to 26%, and preferably ranges from 10 to 18% by weight based on the total weight of the composition. In a preferred composition according to the invention the level of SLES 3EO (i.e. sodium lauryl ether sulphate in which the average degree of ethoxylation n is 3.0) ranges from 10 to 18% by weight based on the total weight of the composition.

The composition of the invention comprises one or more dispersed benefit agents selected from silicones.

The term "benefit agent" in the context of this invention includes materials which can provide a benefit to the hair and/or the scalp and/or the skin (preferably the hair and/or the scalp) as well as those materials which are beneficially incorporated into personal cleansing compositions, such as aesthetic agents.

Hydrophobic emulsion droplets for inclusion in the composition of the invention typically have a mean droplet diameter (D3,2) of 4 micrometres or less. Preferably the mean droplet diameter (D3,2) is 1 micrometre or less, more preferably 0.5 micrometre or less, and most preferably 0.25 micrometre or less.

A suitable method for measuring the mean droplet diameter (D3,2) is by laser light scattering using an instrument such as a Malvern Mastersizer.

Suitable silicones for use in the invention include polydiorganosiloxanes, in particular polydimethylsiloxanes (dimethicones), polydimethyl siloxanes having hydroxyl end groups (dimethiconols), and amino-functional polydimethylsiloxanes (amodimethicones).

Such silicones are preferably non-volatile (with vapour pressure of less than 1000 Pa at 25°C), and preferably have a molecular weight of greater than 100,000, more preferably greater than 250,000.

Such silicones preferably have a kinematic viscosity of greater than 50,000 cS (mm².s⁻¹) and more preferably a kinematic viscosity of greater than 500,000 cS (mm².s⁻¹). Silicone kinematic viscosities in the context of this invention are measured at 25°C and can be measured by means of a glass capillary viscometer as set out further in Dow Corning Corporate Test Method CTM004 July 20, 1970.

Suitable silicones for use in the invention are available as pre-formed silicone emulsions from suppliers such as Dow Corning and GE Silicones. The use of such pre-formed silicone emulsions is preferred for ease of processing and control of silicone particle size. Such pre-formed silicone emulsions will typically additionally comprise a suitable emulsifier, and may be prepared by a chemical emulsification process such as emulsion polymerisation, or by mechanical emulsification using a high shear mixer. Pre-formed silicone emulsions having a mean droplet diameter (D3,2) of less than 0.15 micrometres are generally termed microemulsions.

Examples of suitable pre-formed silicone emulsions include emulsions DC2-1766, DC2-1784, DC-1785, DC-1786, DC-1788, DC-1310, DC-7123 and microemulsions DC2-1865 and DC2-1870, all available from Dow Corning. These are all emulsions/microemulsions of dimethiconol. Also suitable are amodimethicone emulsions such as DC939 (from Dow Corning) and SME253 (from GE Silicones).

Mixtures of any of the above described silicone emulsions may also be used.

In a typical composition according to the invention the level of dispersed benefit agent (as defined above) depends on the particular material(s) used, but generally ranges from 0.01 to 20%, preferably from 0.02 to 10% by weight based on the total weight of the composition. In preferred compositions according to the invention, the one or more dispersed benefit agents includes one or more silicone emulsions (as further described above) and the level of silicone (*per se* as active ingredient) ranges from 0.01 to 10%, preferably from 0.5 to 5% by weight based on the total weight of the composition.

The composition of the invention comprises a structuring polymer selected from alkali swellable emulsion (ASE) polymers and hydrophobically modified alkali swellable emulsion (HASE) polymers.

ASE polymers are carboxyl-containing copolymers that are prepared by the addition polymerization of ethylenically unsaturated monomers. The ASE polymers are insoluble in water at low pH, but exhibit chain expansion and concomitant dissolution at pH greater than 6 upon neutralization with a base.

Exemplary ASE polymers for inclusion in the composition of the invention include linear or crosslinked copolymers that are prepared by the addition polymerization of a monomer mixture including at least one acidic vinyl monomer, such as methacrylic acid or acrylic acid, and at least one nonionic vinyl monomer, such as alkyl acrylate or alkyl methacrylate.

Preferred ASE polymers for inclusion in the composition of the invention are linear or crosslinked copolymers of methacrylic acid and C₁-C₄ alkyl acrylate.

The methacrylic acid is preferably present in the copolymer at from 20 to 80%, more preferably from 25 to 70%, and most preferably from 35 to 65% by weight based on the total weight of the copolymer.

The C₁-C₄ alkyl acrylate is preferably present in the copolymer at from 25 to 70%, and more preferably from 35 to 65% by weight based on the total weight of the copolymer.

The C₁-C₄ alkyl acrylate is preferably selected from methyl acrylate, ethyl acrylate, butyl acrylate or mixtures thereof.

The copolymer is preferably partially or completely crosslinked with at least one ethylenically polyunsaturated crosslinker.

HASE polymers are ASE polymers which have been hydrophobically modified by the incorporation of pendant hydrophobic groups. The HASE polymers operate by a twin mechanism of hydrodynamic thickening, plus association of the hydrophobic groups on the polymer backbone with other hydrophobic species.

Exemplary HASE polymers for inclusion in the composition of the invention include linear or crosslinked copolymers that are prepared by the addition polymerization of a monomer mixture including at least one acidic vinyl monomer, such as methacrylic acid or acrylic acid, and at least one associative monomer.

The term "associative monomer" in the context of this invention denotes a monomer having an ethylenically unsaturated section (for addition polymerization with the other monomers in the mixture) and a hydrophobic section.

In one preferred type of associative monomer, the hydrophobic section is constituted by a homopolymeric, random copolymeric or block copolymeric chain formed from repeating units selected from C₁-C₂₂ alkyl acrylates, C₁-C₂₂ alkyl methacrylates, methacrylic acid, acrylic acid or combinations thereof. Examples of such units include methyl methacrylate, ethyl methacrylate, butyl methacrylate, ethylhexyl methacrylate, stearylmethacrylate and mixtures thereof. One of the units at an end of the chain will remain available as an ethylenically unsaturated section for addition polymerisation with other monomers (as described above). Associative monomers of this type are usually referred to as "macromonomers", and may be prepared by catalytic chain transfer (CCT) procedures utilizing catalysts effective to achieve CCT such as the cobalt porphyrins and the cobaloximes.

Macromonomers may advantageously have a number average molecular weight (Mn as determined by liquid permeation chromatography) ranging from about 200 to about 50,000, preferably from about 400 to about 10,000, and optimally from about 500 to about 3,000.

Particularly preferred examples of macromonomers formed from the sections described above include poly(methylmethacrylate)/poly(methacrylic acid), poly(methylmethacrylate), poly(butylmethacrylate), poly(ethylhexylmethacrylate) and combinations thereof.

Another preferred type of associative monomer includes a polyoxyalkylene section between the ethylenically unsaturated section and the hydrophobic section. Associative monomers of this type are sometimes referred to as "surfmers", and may typically be prepared by the acid catalyzed condensation of commercially available nonionic polyoxyalkylene surfactant alcohols with acrylic, methacrylic, crotonic, maleic, fumaric, itaconic or aconitic acid.

The ethylenically unsaturated section of the surfmer is typically derived from an alpha, beta-ethylenically unsaturated mono or dicarboxylic acid or the anhydride thereof, more preferably a C₃-C₄ mono- or dicarboxylic acid or the anhydride thereof. Alternatively, the ethylenically unsaturated section can be derived from an allyl ether or a vinyl ether, a nonionic vinyl-substituted urethane monomer or a vinyl-substituted urea reaction product. The polyoxyalkylene section of the surfmer is suitably a homopolymeric or a random or block copolymeric chain formed from about 5 to about 250, more preferably from about 10 to about 120, and most preferably from about 15 to about 60 repeating oxy (C₂-C₄ alkylene) units. Preferred polyoxyalkylene sections include polyoxyethylene, polyoxypropylene, and polyoxybutylene sections formed from about 5 to about 150, more preferably from about 10 to about 100 and most preferably from about 15 to about 60 oxyethylene, oxypropylene or oxybutylene units respectively.

The hydrophobic section of the surfmer is preferably a hydrocarbyl group belonging to one of the following classes: C₈-C₄₀ linear alkyl, aryl-substituted C₂-C₄₀ alkyl, C₂-C₄₀ alkylsubstituted phenyl, C₈-C₄₀ branched alkyl, C₈-C₄₀ carbocyclic alkyl; and Cs-Cso complex ester.

Preferred examples of such hydrophobic sections include linear or branched C₈-C₄₀ alkyl groups such as capryl, isooctyl, decyl, lauryl, myristyl, cetyl, cetearyl, stearyl, isostearyl, arachidyl, behenyl and mixtures thereof; and Cs-Cso complex esters such as hydrogenated castor oil (predominately the glyceride of 12-hydroxystearic acid), 1,2-diacyl glycerols (such as 1,2-distearyl glycerol, 1,2-dipalmityl glycerol and 1,2-dimyristyl glycerol), di-, tri-, or polymers of sugars (such as 3,4,6-tristearyl glucose and 2,3-dilauryl fructose) and sorbitan esters.

Particularly preferred examples of surfmers formed from the sections described above include cetyl polyethoxylated methacrylate, cetearyl polyethoxylated methacrylate, stearyl polyethoxylated (meth)acrylate, arachidyl polyethoxylated (meth)acrylate, behenyl polyethoxylated methacrylate, lauryl polyethoxylated methacrylate, hydrogenated castor oil polyethoxylated methacrylate, and canola polyethoxylated (meth)acrylate, wherein the polyethoxylated portion comprises about 5 to about 100, preferably about 10 to about 80, and more preferably about 15 to about 60 oxyethylene repeating units.

Preferred HASE polymers for use in the invention includes linear or crosslinked copolymers of methacrylic acid or acrylic acid with (i) at least one associative monomer selected from surfmers (as defined above), macromonomers (as defined above) and mixtures thereof; and (ii) at least one further monomer selected from C₁-C₄ alkyl acrylates or methacrylates, polyacidic vinyl monomers and mixtures thereof.

The methacrylic acid or acrylic acid is preferably present in the copolymer at from 10 to 80%, more preferably from 25 to 70%, and most preferably from 35 to 65% by weight based on the total weight of the copolymer.

The associative monomer is preferably present in the copolymer at from 0.5 to 25%, more preferably from 0.5 to 15% by weight based on the total weight of the copolymer.

The C₁-C₄ alkyl acrylate or methacrylate is preferably selected from methyl acrylate, ethyl acrylate, butyl acrylate or mixtures thereof.

When present, the amount of C₁-C₄ alkyl acrylate or methacrylate in the copolymer may preferably range from 25 to 85%, and more preferably from 35 to 65%

The polyacidic vinyl monomer is preferably selected from maleic, fumaric, itaconic and citraconic acids, anhydrides and salts thereof and mixtures thereof. More preferred are maleic acid, maleic anhydride and salts thereof and mixtures thereof.

When present, the amount of polyacidic vinyl monomer may preferably range from 0.1 to about 10% by weight based on the total weight of the copolymer.

Specific examples of HASE polymers for inclusion in the composition of the invention include copolymers of acrylic acid with alkyl acrylates with the INCI name Acrylates Copolymer (e.g. Aculyn® 33 from Rohm & Haas), copolymers of acrylic acid with ethyl acrylate and associative alkyl acrylates with the INCI name Acrylates Copolymer (Carbopol Aqua SF-1® from Lubrizol) or copolymers of (meth)acrylic acid with alkyl acrylates and ethoxylated hydrophobically modified alkyl acrylates with the INCI names Acrylates/Steareth-20 Methacrylate Copolymer, Acrylates/Beheneth-25 Methacrylate Copolymer, Acrylates/Steareth-20 Methacrylate Crosspolymer (Aculyn® 22, 28 or 88 from Rohm & Haas) or the INCI name Acrylates/Palmeth-25 Acrylates Copolymer (Synthalen® from 3V Sigma).

Mixtures of any of the above described materials may also be used.

Preferably the structuring polymer is selected from HASE polymers as described above.

The level of structuring polymer depends on the particular material(s) used, but generally ranges from 0.1 % to 1.5% by weight based on the total weight of the composition.

The composition of the invention may suitably include at least one inorganic electrolyte. The inorganic electrolyte may be used to help provide viscosity to the composition.

The viscosity of the composition suitably ranges from 6,000 to 10,000 mPa.s, preferably from 7,000 to 9,000 mPa.s, more preferably from 7,500 to 8,500 mPa.s when measured using a Brookfield V2 viscometer (spindle RTV5, 1 minute, 20rpm) at 30°C.

Suitable inorganic electrolytes include metal chlorides (such as sodium chloride, potassium chloride, calcium chloride, magnesium chloride, zinc chloride, ferric chloride and aluminium chloride) and metal sulphates (such as sodium sulphate and magnesium sulphate).

Examples of preferred inorganic electrolytes for use in the invention include sodium chloride, potassium chloride, magnesium sulphate and mixtures thereof.

Mixtures of any of the above described materials may also be suitable.

The level of inorganic electrolyte in compositions of the invention generally ranges from about 1 to about 25%, preferably from about 2 to about 20%, more preferably from about 3 to about 15% (by total weight inorganic electrolyte based on the total weight of the composition).

The composition of the invention preferably includes one or more cationic polymers. Such polymers may enhance the delivery of conditioning agents and thereby improve the conditioning benefits obtained.

Cationic polymers typically contain cationic nitrogen-containing groups such as quaternary ammonium or protonated amino groups. The protonated amino groups can be primary, secondary, or tertiary amines (preferably secondary or tertiary). The cationic nitrogen-containing group of the cationic polymer is generally present as a substituent on all, or more typically on some, of the repeat units making up the polymer.

Preferably the cationic nitrogen-containing groups are selected from quaternary ammonium groups bearing three radicals, which may be identical or different, chosen from hydrogen and alkyl radicals containing 1 to 10, more preferably 1 to 6 and most preferably 1 to 3 carbon atoms.

Any anionic counterion can be used in association with the cationic polymer so long as the counterion is physically and chemically compatible with the essential components of the composition or does not otherwise unduly impair product performance, stability or aesthetics. Examples of such counterions include halides (e.g., chloride, fluoride, bromide, iodide), sulfate and methylsulfate.

The weight average molecular weight (Mw) of the cationic polymer is preferably from about 5,000 to about 10 million, more preferably from about 100,000 to about 1 million g/mol.

The cationic polymer generally has a cationic charge density ranging from about 0.2 to about 7 meq/gm. The term "cationic charge density" in the context of this invention refers to the ratio of the number of positive charges on a monomeric unit of which a polymer is comprised to the molecular weight of the monomeric unit. The charge density multiplied by the polymer molecular weight determines the number of positively charged sites on a given polymer chain. Cationic charge density can be determined according to the Kjeldahl Method. Those skilled in the art will recognize that the charge density of amino-containing polymers may vary depending upon pH and the isoelectric point of the amino groups. The charge density should be within the above limits at the pH of intended use. Cationic polymers for use in the invention preferably have a cationic charge density ranging from about 0.3 to about 4 meq/g, more preferably from about 0.4 to about 3.5 meq/g.

Suitable cationic polymers for use in the invention include cationic polysaccharide derivatives, such as cationic cellulose derivatives, cationic starch derivatives, and cationic guar gum derivatives.

Preferred cationic polysaccharide derivatives for use in the invention include cationic guar gum derivatives and cationic cellulose derivatives.

Examples of preferred cationic guar gum derivatives for use in the invention include guar hydroxypropyltrimethylammonium chlorides such as JAGUAR ® C13S, JAGUAR ® C14, JAGUAR® C15 and JAGUAR ® C17.

Examples of preferred cationic cellulose derivatives for use in the invention include poly(1,2-oxyethanediyl)-2-hydroxy-3-trimethylammonium propyl chloride cellulose ethers (INCI: Polyquaternium-10) such as UCARE® Polymer JR-125, UCARE® Polymer JR-400, UCARE® Polymer JR-30M, UCARE® Polymer LR-400 and UCARE® Polymer LR-30M.

Mixtures of any of the above described cationic polymers may also be used.

When included, the total level of cationic polymer in the composition is preferably from 0.05% to 2% and more preferably from 0.1 to 0.5% by weight based on the total weight of the composition.

The composition of the invention preferably includes one or more amphoteric surfactants. Suitable amphoteric surfactants are betaines, such as those having the general formula R(CH₃)₂N⁺CH₂COO⁻, where R is an alkyl or alkylamidoalkyl group, the alkyl group preferably having 10 to 16 carbon atoms. Particularly suitable betaines are oleyl betaine, caprylamidopropyl betaine, lauramidopropyl betaine, isostearylamidopropyl betaine, and cocoamidopropyl betaine.

When included, the total level of amphoteric surfactant is generally from 0.1% to 20%, preferably from 1% to 10%, more preferably from 1% to 5% by weight based on the total weight of the composition.

A mildly acidic pH is often desirable in shampoos for the reasons quoted above. Advantageously, the present invention provides a solution to the problem of inefficient deposition of dispersed benefit agents (such as silicones), thus allowing the composition of the invention to be formulated at lower pH values.

Mildly acidic compositions of the invention as described above will suitably have a pH ranging from about 3.8 to about 5.4, preferably from about 3.9 to about 5.2 , more preferably from about 4.0 to about 5.1 and most preferably from about 4.1 to 4.9 The pH of such a composition may be adjusted to the desired value using any cosmetically acceptable organic or inorganic acid, such as citric acid, acetic acid, glycolic acid, lactic acid, malic acid, tartaric acid, hydrochloric acid and mixtures thereof.

A composition of the invention may contain further optional ingredients to enhance performance and/or consumer acceptability. Examples of such ingredients include fragrance, dyes and pigments, pH adjusting agents and preservatives or antimicrobials. Each of these ingredients will be present in an amount effective to accomplish its purpose. Generally these optional ingredients are included individually at a level of up to 5% by weight based on the total weight of the composition.

One preferred class of optional ingredient for use in the invention includes organic acid preservatives. Suitable organic acid preservatives may be selected from benzoic acid and/or alkali metal and ammonium salts thereof (e.g. sodium benzoate); sorbic acid and/or alkali metal and ammonium salts thereof (e.g. potassium sorbate); p-anisic acid and/or alkali metal and ammonium salts thereof, and salicylic acid and/or alkali metal and ammonium salts thereof. Mixtures of any of the above-described ingredients may also be used. Preferred organic acid preservatives are selected from benzoic acid/sodium benzoate, sorbic acid/potassium sorbate, or mixtures thereof. Most preferred is benzoic acid/sodium benzoate.

When included, the total level of organic acid preservative (as described above) generally ranges from about 0.01% to about 3%, preferably from about 0.05% to about 2%, more preferably from about 0.1% to about 1% by weight based on the total weight of the composition.

In a composition of the invention including organic preservative as described above, it is preferred that the composition pH is mildly acidic (as is further described above), to ensure that the organic acid preservative is present in its active protonated form.

### Process

The invention also provides a process of manufacturing a personal cleansing composition as defined above, the process comprising the steps of swelling the structuring polymer (iii) in an aqueous solution and combining the swollen structuring polymer so obtained with the remaining composition ingredients.

Preferably the structuring polymer is swollen by dissolving the polymer in water and increasing the pH until no further change in viscosity occurs. The pH may be readjusted as necessary after combination of the swollen structuring polymer with the remaining composition ingredients, so that the pH of the final composition ranges from 3 to 6.5.

### Mode of Use

The composition of the invention is primarily intended for topical application to the body, preferably the hair and scalp.

Most preferably the composition of the invention is topically applied to the hair and then massaged into the hair and scalp. The composition is then rinsed off the hair and scalp with water prior to drying the hair.

The invention will be further illustrated by the following, non-limiting Examples, in which all percentages quoted are by weight based on total weight unless otherwise stated.

### EXAMPLES

Hair cleansing shampoo formulations were prepared, having ingredients as shown in Table 1 below. Examples 1 to 4 represent formulations according to the invention. Examples A to D represent comparative examples (not according to the invention).

**Table 1**

| **Ingredient** | **Example A** | **Example B** | **Example 1** | **Example 2** |
|---|---|---|---|---|
| | wt% | | | |
| Sodium laureth sulfate (1EO) | 12 | 12 | - | - |
| Sodium laureth sulfate (3EO) | - | - | 12 | 12 |
| Cocamidopropyl betaine | 1.6 | 1.6 | 1.6 | 1.6 |
| HASE polymer | 0.4 | 0.4 | 0.4 | 0.4 |
| Citric acid | To pH 6.5 | To pH 6.5 | To pH 6.5 | To pH 6.5 |
| Ethylene glycol distearate | 2 | 2 | 2 | 2 |
| Guar hydroxypropyltrimonium chloride (JAGUAR® C13S) | 0.2 | - | 0.2 | - |
| Polyquaternium-10 | - | 0.2 | - | 0.2 |
| Preservative | 0.5 | 0.5 | 0.5 | 0.5 |
| Salt | 1 | 1 | 1 | 1 |
| Disodium EDTA | 0.05 | 0.05 | 0.05 | 0.05 |
| Perfume | 0.7 | 0.7 | 0.7 | 0.7 |
| Polypropylene glycol | 0.15 | 0.15 | 0.15 | 0.15 |
| Silicone | 0.63 | 0.63 | 0.63 | 0.63 |
| Mica | 0.2 | 0.2 | 0.2 | 0.2 |
| Glycerin | 0.5 | 0.5 | 0.5 | 0.5 |
| Water | q.s.to 100 | q.s.to 100 | q.s.to 100 | q.s.to 100 |

| **Ingredient** | **Example C** | **Example D** | **Example 3** | **Example 4** |
|---|---|---|---|---|
| | wt% | | | |
| Sodium laureth sulfate (1EO) | 12 | 12 | - | - |
| Sodium laureth sulfate (3EO) | - | - | 12 | 12 |
| Cocamidopropyl betaine | 1.6 | 1.6 | 1.6 | 1.6 |
| HASE polymer | 0.4 | 0.4 | 0.4 | 0.4 |
| Citric acid | To pH 4.5 | To pH 4.5 | To pH 4.5 | To pH 4.5 |
| Ethylene glycol distearate | 2 | 2 | 2 | 2 |
| Guar hydroxypropyltrimonium chloride (JAGUAR® C13S) | 0.2 | - | 0.2 | - |
| Polyquaternium-10 | - | 0.2 | - | 0.2 |
| Preservative | 0.5 | 0.5 | 0.5 | 0.5 |
| Salt | 1 | 1 | 1 | 1 |
| Disodium EDTA | 0.05 | 0.05 | 0.05 | 0.05 |
| Perfume | 0.7 | 0.7 | 0.7 | 0.7 |
| Polypropylene glycol | 0.15 | 0.15 | 0.15 | 0.15 |
| Silicone | 0.63 | 0.63 | 0.63 | 0.63 |
| Mica | 0.2 | 0.2 | 0.2 | 0.2 |
| Glycerin | 0.5 | 0.5 | 0.5 | 0.5 |
| Water | q.s.to 100 | q.s.to 100 | q.s.to 100 | q.s.to 100 |

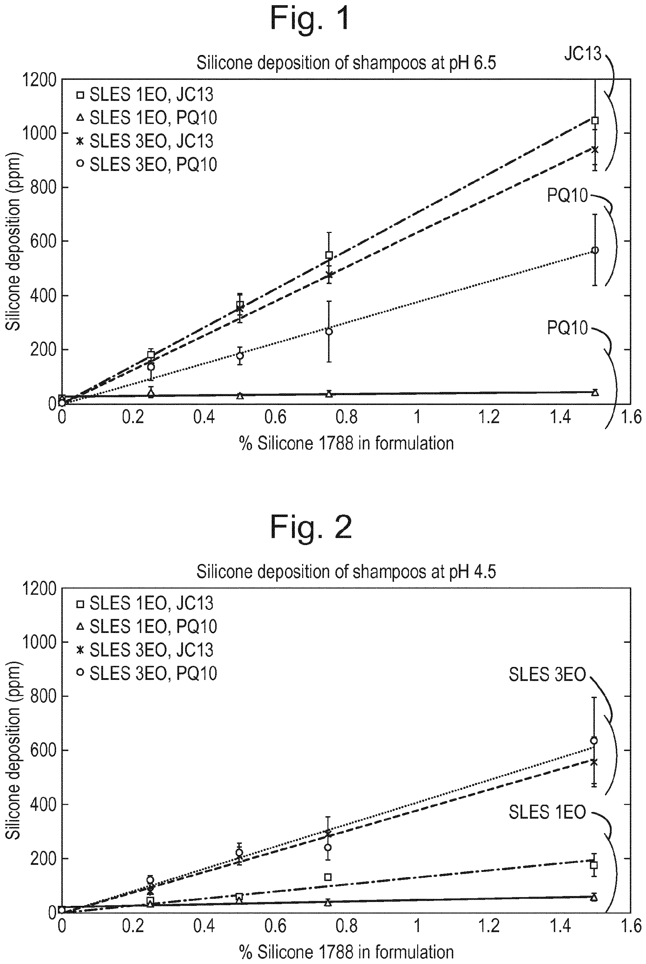

Hair switches were treated with the shampoo products and silicone deposition was measured using XRF (standard protocol). The results for Examples A, B, 1 and 2 (shampoo pH 6.5) are shown in Figure 1. The results for Examples C, D, 3 and 4 (shampoo pH 4.5) are shown in Figure 2.

Referring to Figure 1, silicone deposition values for the comparative formulations using SLES (1EO) drop to virtually zero when the cationic polymer JAGUAR® C13S (JC13) is replaced with the cationic polymer Polyquaternium-10 (PQ-10).

Referring to Figure 2, silicone deposition values are poor for the comparative formulations using SLES (1EO) regardless of the cationic polymer used. For the inventive formulations using SLES (3EO), silicone deposition is 3 times better than the comparative formulations with JAGUAR® C13S and also with Polyquaternium-10.

From a comparison of Figures 1 and 2, it can be seen that SLES (3EO) formulations according to the invention deliver good levels of silicone deposition with Polyquaternium-10 (about 600 ppm), and the deposition performance is not dependent on the formulation pH.

In summary, the results show that the comparative formulations using SLES (1EO) exhibit silicone deposition values which are extremely sensitive to both formulation pH and the nature of the cationic polymer used. By contrast, the inventive formulations using SLES (3EO) exhibit good silicone deposition values which are tolerant to both formulation pH and the nature of the cationic polymer used.

## Claims

1. A personal cleansing composition having a pH ranging from 4.1 to 4.9 and comprising, in an aqueous continuous phase:
(i) one or more anionic cleansing surfactants;
(ii) one or more dispersed benefit agents selected from silicones, and
(iii) a structuring polymer selected from alkali swellable emulsion (ASE) polymers and hydrophobically modified alkali swellable emulsion (HASE) polymers;
**characterized in that** the one or more anionic cleansing surfactants are selected from alkyl ether sulphates of general formula (I):
R-O-(CH₂CH₂-O)ₙ-SO₃⁻M⁺ (I)
in which R is a straight or branched chain alkyl group having 10 to 14 carbon atoms,
n is a number that represents the degree of ethoxylation and ranges from 3 to 3.5,
and M is a alkali metal, ammonium or alkanolammonium cation.

2. A composition according to claim 1, in which in general formula (I), M is sodium, potassium, ammonium or ethanolamine, R is a C₁₀ tor C₁₂ *n*-alkyl group and n ranges from 3.0 to 3.2.

3. A composition according to claim 1 or claim 2, in which the structuring polymer (iii) is a HASE polymer selected from linear or crosslinked copolymers that are prepared by the addition polymerization of a monomer mixture including at least one acidic vinyl monomer and at least one associative monomer.

4. A composition according to claim 3, in which the HASE polymer is selected from linear or crosslinked copolymers of methacrylic acid or acrylic acid with (i) at least one associative monomer selected from surfmers which comprises polyoxyalkylene section between the ethylenically unsaturated section and the hydrophobic section, macromonomers and mixtures thereof; and (ii) at least one further monomer selected from C₁-C₄ alkyl acrylates or methacrylates, polyacidic vinyl monomers and mixtures thereof.

5. A composition according to claim 4, in which the associative monomer is a surfmer selected from cetyl polyethoxylated methacrylate, cetearyl polyethoxylated methacrylate, stearyl polyethoxylated (meth)acrylate, arachidyl polyethoxylated (meth)acrylate, behenyl polyethoxylated methacrylate, lauryl polyethoxylated methacrylate, hydrogenated castor oil polyethoxylated methacrylate, and canola polyethoxylated (meth)acrylate, wherein the polyethoxylated portion comprises from 15 to 60 oxyethylene repeating units.

6. A composition according to claim 4, in which the associative monomer is a macromonomer, having a number average molecular weight (Mn as determined by liquid permeation chromatography) ranging from 500 to 3,000, and selected from poly(methylmethacrylate)/poly(methacrylic acid), poly(methylmethacrylate), poly(butylmethacrylate), poly(ethylhexylmethacrylate) and combinations thereof.

7. A composition according to any preceding claim, in which the one or more dispersed benefit agents (ii) includes one or more silicone emulsions and the level of silicone (*per se* as active ingredient) ranges from 0.5 to 5% by weight based on the total weight of the composition.

## Patentansprüche

1. Körperreinigungszusammensetzung, die einen von 4,1 bis 4,9 reichenden pH-Wert aufweist und in einer wässrigen kontinuierlichen Phase umfasst:
(i) ein oder mehrere anionische Reinigungstensid(e);
(ii) ein oder mehrere dispergierte Vorteilsmittel, ausgewählt aus Siliconen und
(iii) ein strukturgebendes Polymer, ausgewählt aus Alkali-quellbaren Emulsions (ASE)-Polymeren und hydrophob modifizierten Alkali-quellbaren Emulsions (HASE)-Polymeren;
**dadurch gekennzeichnet, dass** das eine oder die mehreren anionische(n) Reinigungstensid(e) aus Alkylethersulfaten der allgemeinen Formel (I):
R-O-(CH₂CH₂-O)ₙ-SO₃-M⁺ (I)
ausgewählt ist/sind,
in welcher R eine gerad- oder verzweigtkettige Alkylgruppe, die 10 bis 14 Kohlenstoffatome aufweist, ist, n eine Zahl ist, die den Grad der Ethoxylierung darstellt und von 3 bis 3,5 reicht, und M ein Alkalimetall-, Ammonium- oder Alkanolammonium-Kation darstellt.

2. Zusammensetzung nach Anspruch 1, in welcher in der allgemeinen Formel (I) M Natrium, Kalium, Ammonium oder Ethanolamin ist, R eine C₁₀- bis C₁₂-n-Alkylgruppe ist und n von 3,0 bis 3,2 reicht.

3. Zusammensetzung nach Anspruch 1 oder Anspruch 2, in welcher das strukturgebende Polymer (iii) ein HASE-Polymer ist, ausgewählt aus linearen oder vernetzten Copolymeren, die durch die Additionspolymerisation einer Monomermischung, einschließlich mindestens eines sauren Vinylmonomers und mindestens eines assoziativen Monomers, hergestellt werden.

4. Zusammensetzung nach Anspruch 3, in welcher das HASE-Polymer ausgewählt ist aus linearen oder vernetzten Copolymeren von Methacrylsäure oder Acrylsäure mit (i) mit mindestens einem assoziativen Monomer, ausgewählt aus Surfmeren, die einen Polyoxyalkylen-Abschnitt zwischen dem ethylenisch ungesättigten Abschnitt und dem hydrophoben Abschnitt umfassen, Makromonomeren und Mischungen davon; und (ii) mindestens einem weiteren Monomer, ausgewählt aus C₁-C₄-Alkylacrylaten oder -Methacrylaten, mehrfach sauren Vinylmonomeren und Mischungen davon.

5. Zusammensetzung nach Anspruch 4, in welcher das assoziative Monomer ein Surfmer darstellt, ausgewählt aus Cetyl-polyethoxyliertem (Meth)acrylat, Cetearylpolyethoxyliertem (Meth)acrylat, Stearyl-polyethoxyliertem (Meth)acrylat, Arachidyl-polyethoxyliertem (Meth)acrylat, Behenyl-polyethoxyliertem (Meth)acrylat, Lauryl-polyethoxyliertem (Meth)acrylat, hydriertes Rizinusölpolyethoxyliertem (Meth)acrylat und Canola-polyethoxyliertem (Meth)acrylat, wobei der polyethoxylierte Teil von 15 bis 60 Oxyethylen-Wiederholungseinheiten umfasst.

6. Zusammensetzung nach Anspruch 4, in welcher das assoziative Monomer ein Makromonomer ist, das ein zahlenmittleres Molekulargewicht (Mn, bestimmt durch Flüssig-Permeationschromatographie) aufweist, das von 500 bis 3.000 reicht, und aus Poly(methylmethacrylat)/Poly(methacrylsäure), Poly(methylmethacrylat), Poly(butylmethacrylat), Poly(ethylhexylmethacrylat) und Kombinationen davon ausgewählt ist.

7. Zusammensetzung nach irgendeinem vorhergehenden Anspruch, in welcher das eine oder die mehreren dispergierten Vorteilsmittel (ii) ein oder mehrere Silicon-Emulsionen einbeziehen und der Anteil des Silicons (per se als aktiver Bestandteil) von 0,5 bis 5 Gew.-%, bezogen auf das Gesamtgewicht der Zusammensetzung, reicht.

## Revendications

1. Composition nettoyante pour la personne ayant un pH de 4,1 à 4,9 et comprenant, dans une phase continue aqueuse :
(i) un ou plusieurs tensioactifs nettoyants anioniques ;
(ii) un ou plusieurs agents bénéfiques dispersés choisis parmi des silicones, et
(iii) un polymère structurant choisi parmi des polymères en émulsion gonflables par un alcali (ASE) et des polymères en émulsion gonflables par un alcali hydrophiquement modifiés (HASE) ;
**caractérisée en ce que** les uns ou plusieurs tensioactifs nettoyants anioniques sont choisis parmi des sulfates d'alkyléthers de formule générale (I):
R-O-(CH₂CH₂-O)ₙ-SO₃⁻M⁺ (I)
dans laquelle R est un groupe alkyle à chaîne linéaire ou ramifiée ayant de 10 à 14 atomes de carbone,
n est un nombre qui représente le degré d'éthoxylation et est de 3 à 3,5,
et M est un métal alcalin, un cation ammonium ou alcanolammonium.

2. Composition selon la revendication 1, dans laquelle dans la formule générale (I), M est le sodium, le potassium, l'ammonium ou l'éthanolamine, R est un groupe *n*-alkyle en C₁₀ à C₁₂ et n est de 3,0 à 3,2.

3. Composition selon la revendication 1 ou la revendication 2, dans laquelle le polymère structurant (iii) est un polymère HASE choisi parmi des copolymères linéaires ou réticulés qui sont préparés par la polymérisation par addition d'un mélange de monomères incluant au moins un monomère vinylique acide et au moins un monomère associatif.

4. Composition selon la revendication 3, dans laquelle le polymère HASE est choisi parmi des copolymères linéaires ou réticulés d'acide méthacrylique ou d'acide acrylique avec (i) au moins un monomère associatif choisi parmi des surfmères qui comprennent une section polyoxyalkylène entre la section éthyléniquement insaturée et la section hydrophobe, des macromonomères et des mélanges de ceux-ci ; et (ii) au moins un autre monomère choisi parmi des acrylates ou méthacrylates d'alkyle en C₁-C₄, des monomères vinyliques polyacides et des mélanges de ceux-ci.

5. Composition selon la revendication 4, dans laquelle le monomère associatif est un surfmère choisi parmi un méthacrylate polyéthoxylé de cétyle, méthacrylate polyéthoxylé de cétéaryle, (méth)acrylate polyéthoxylé de stéaryle, (méth)acrylate polyéthoxylé d'arachidyle, méthacrylate polyéthoxylé de béhényle, méthacrylate polyéthoxylé de lauryle, méthacrylate polyéthoxylé d'huile de ricin hydrogénée, et (méth)acrylate polyéthoxylé de canola, dans laquelle la portion polyéthoxylée comprend de 15 à 60 unités répétitives oxyéthylène.

6. Composition selon la revendication 4, dans laquelle le monomère associatif est un macromonomère, ayant une masse moléculaire moyenne en nombre (Mn comme déterminée par chromatographie par perméation liquide) de 500 à 3 000, et choisi parmi le poly(méthacrylate de méthyle)/poly(acide méthacrylique), poly(méthacrylate de méthyle), poly(méthacrylate de butyle), poly(méthacrylate d'éthyhexyle) et des combinaisons de ceux-ci.

7. Composition selon l'une quelconque des revendications précédentes, dans laquelle les uns ou plusieurs agents bénéfiques dispersés (ii) comprennent une ou plusieurs émulsions de silicone et la teneur en silicone (*per se* comme ingrédient actif) est de 0,5 à 5 % en masse rapporté à la masse totale de la composition.
